Europäisches Patentamt

European Patent Office

Office européen des brevets

(11) Publication number: **0 102 495**
**A2**

## (12) EUROPEAN PATENT APPLICATION

(21) Application number: **83107120.4**

(22) Date of filing: **21.07.83**

(51) Int. Cl.[3]: **A 61 K 7/09**

(30) Priority: **30.07.82 US 401881**

(43) Date of publication of application:
**14.03.84 Bulletin 84/11**

(84) Designated Contracting States:
**AT BE DE FR GB IT**

(71) Applicant: **Helene Curtis Industries, Inc.**
**4401 West North Avenue**
**Chicago, Ill. 60639(US)**

(72) Inventor: **Hsiung, Du Yung**
**130 Illinois Street**
**Park Forest Illinois 60 466(US)**

(74) Representative: **Reitzner, Bruno, Dr.**
**Patentanwälte Dipl.-Ing. R. Splanemann Dr. B. Reitzner**
**Tal 13**
**D-8000 München 2(DE)**

(54) Hair waving system and lotion, and method of waving hair.

(57) A hair waving system comprising at least two separately packaged components and methods of its use are described. One of the components is an aqueous activator containing about 0.5 to about 12 molar urea stabilized with a sufficient amount of an ammonium salt of a strong acid to adjust the pH value of the activator to about 6-8. The second package of the system contains an amount of hair disulfide bond-breaking agent sufficient to wave hair after admixture with the activator, and provides a pH value of 6.5-9.5 to the admixed waving lotion. A particular waving lotion is also disclosed.

EP 0 102 495 A2

Croydon Printing Company Ltd

4401 West North Avenue
Chicago, Illinois, 60 639
U S A

0102495

Hair Waving System and Lotion,
and Method of Waving Hair

Technical Field

The present invention relates to hair waving compositions that contain urea or a substituted urea, and more particularly to an aqueous hair waving composition and method in which the urea or the substituted urea is relatively stable to hydrolysis upon aging of the composition.

Background Art

Urea and lower alkyl-substituted ureas such as N-methylurea are well known in the cosmetic arts, and particularly in the hair waving and straightening arts, as useful agents in promoting the treatment being carried out. For example, U.S. Patent No. 2,717,228 teaches that urea or mono-alkyl substituted ureas can be used at about 0.5 to about 4 molar with thiol compounds that are present at 0.35 to about 0.8 molar in hair waving compositions having a pH value between about 8.6 and about 9.5. This patent teaches that use of the urea permits a reduction in the amount of thiol present while maintaining a given amount of waving. Similarly, U.S. Patent No. 4,243,659 teaches the use of N,N'-dimethylurea as a swelling agent in cleansing compositions that contain the bisulfite ion along with detergent.

The beneficial attributes of the use of urea in cosmetic compositions are tempered by the fact that urea and its lower alkyl derivatives, when dissolved in the aqueous cosmetic composition, tend to hydrolyze on storage to produce an alkali, such as ammonia or a lower alkyl-substituted amine. This hydrolysis of urea is evidenced by the teachings of U.S. Patent No. 3,847,165 wherein it is taught that a

-2-

"two-stage system" is required for the use of urea because of the instability of urea in solution over long periods of time. Similarly, U.S. Patent No. 4,243,659 teaches that cleansing compositions containing bisulfite ion and N,N'-dimethylurea are prepared at a pH in the range of 4.0 to 6.9 "to substantially prevent alkaline hydrolysis of the urea derivative." Still further, U.S. Patent No. 4,177,260 teaches that urea is not stable in aqueous solutions that contain sulfite ions at pH 6 and saponifies to yield ammonium carbonate. The saponification or hydrolysis of urea is said to be avoided in that latter patent by the use of a specific cyclic urea; i.e., ethylene urea or imidazolidinone-(2).

The problems associated with the hydrolysis of urea and its lower alkyl-substituted derivatives are avoided to some extent by the use of separately packaged, dry urea that is mixed into the cosmetic composition just prior to use. An example of the use of dry urea that is dissolved prior to use in a cosmetic composition can be found in U.S. No. 3,847,165.

While the use of dry urea tends to overcome the problem of hydrolysis of the urea, or its derivative, upon storage, that use also has several disadvantages. First, dissolution of a dry component into a solution is time consuming, inconvenient and sometimes incomplete. In addition, dissolution of urea in water is endothermic, leading to a considerable drop in the temperature of the resulting solution, and the use of such cold solutions in a hair treating, cosmetic composition such as in hair waving tends to retard the rate at which waving occurs.

-3-

On the other hand, if the useful urea were in solution and two or more solutions need only to be admixed, that admixture is relatively easily done and will not produce as drastic a change in temperature as when the same amount of solid urea is dissolved to form the same amount of solution.

It is thus seen that if an aqueous solution of urea could be made stable to hydrolysis on storage, such an aqueous solution would be a great benefit to the cosmetics industry.

Summary of the Invention

The present invention relates to hair waving compositions and methods that utilize aqueous compositions of an urea compound and a water-soluble reductive hair disulfide bond-breaking agent.

One embodiment utilizes a stabilized composition of an urea compound as an activator for a waving lotion system. The activator contains water, urea itself or a lower alkyl-substituted urea or mixtures thereof that is present in the solution at about 0.5 to about 12 molar. Also in the activator solution is an amount of an ammonium salt of a strong acid that is sufficient to adjust the pH value of the urea solution to about 6 to about 8. The amount of the ammonium salt of a strong acid that is typically useful in the activator ranges from about 0.02 molar to about 1 molar.

The activator is used as a separately packaged component of a hair waving system. Another separately packaged component is a base lotion which includes an aqueous solution of a water-soluble hair disulfide bond-breaking agent. In this hair waving system, the two, separately packaged liquid components are admixed prior to use to form a waving lotion of this invention. This hair waving system

-4-

can be included in a hair waving product that also contains a separately packaged neutralizer to reform the broken hair disulfide bonds and complete the waving process.

A hair waving lotion of this invention can be prepared from the hair waving system of this invention, although it need not be so prepared. The waving lotion contains water, about 4 to about 25 weight percent urea, a water-soluble hair disulfide bond breaking agent, present at about 4 to about 20 weight percent, and about 0.1 to about 2 weight percent of an ammonium salt of a strong acid after admixture. This waving lotion has a pH value of about 6.5 to about 8.

The present invention also includes methods in which both the waving lotion and hair waving system are utilized.

The present invention has several benefits and advantages. One advantage is that it provides aqeuous urea solutions that exhibit reduced amounts of titratable alkali that results from urea hydrolysis on aging at elevated temperatures.

Particularly beneficial is the presence of both urea and an ammonium salt of a strong acid in a waving lotion having a pH value of about 6.5 to about 8 and containing ammonium thioglycolate as the hair disulfide bond-breaking agent. This waving lotion provides a noticeably higher curl level and/or curl spring then does a waving lotion containing the same pH value and amounts of ammonium thioglycolate and urea which does not contain an ammonium salt of a strong acid.

Another benefit of the present invention is that the use of urea in waving can be obtained without the disadvantage of the urea being supplied as a solid or as a more costly cyclic compound.

-5-

Still further benefits and advantages of the present invention will become apparent to those skilled in the art from the detailed description of the invention and in the claims which follow.

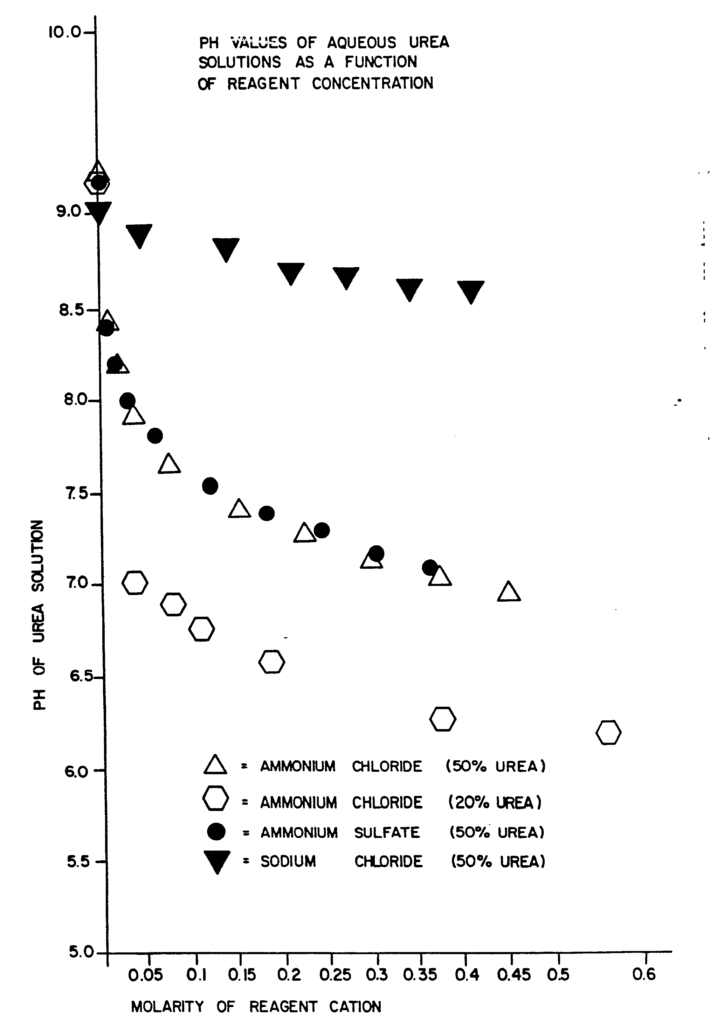

Brief Description of the Drawing

In the drawing, forming a portion of the disclosure of this invention;

The sole Figure is a graph showing the effect upon the pH value of aqueous urea solutions caused by the addition of differing amounts of reagents.

Detailed Description of the Invention

Urea and lower alkyl-substituted ureas are usually considered to be neither acids nor bases, but neutral compounds. However, because ureas are particularly susceptible to hydrolysis, dissolution of urea and lower alkyl-substituted ureas in water produces aqueous solutions having a basic pH value. For example, aqueous solutions that contain about 50 weight percent urea can have pH values as high as about 9 to about 10, probably because of hydrolysis of solid urea in the presence of moist air, as is shown in the graph of the Figure.

Dissolved in aqueous solution, urea and lower alkyl-substituted ureas hydrolyze substantially in a few weeks, and cannot therefore be used as such in commercial cosmetic products. It has now surprisingly been found that an activator containing an aqueous solution of urea, lower alkyl-substituted ureas or mixtures thereof can be stabilized to hydrolysis for extended periods of time, even when stored at elevated temperatures, by the presence of an ammonium salt of a strong acid that is present in an amount sufficient to adjust the pH value of the resulting solution to about 6 to about 8.

-6-

The phrases "stable", "stable to hydrolysis" and "stable to the production of titratable alkali" and the various grammatical forms of the words of those phrases are used herein to mean that the solution so characterized, which has a pH value of about 6 to about 8 and contains a urea compound and an ammonium salt of a strong acid, produces about one-half or less of the amount of titratable alkali than does a similar composition that contains the same amount of urea, but is substantially free from an ammonium salt of a strong acid after both solutions are stored at 43°C. for a period of two months. A method for determining the amount of titratable alkali is shown in Example 1. The amount of titratable alkali produced is calculated by subtracting the amount of titratable alkali initially present from the amount found after the storage period.

The urea compounds useful in a waving lotion activator and waving lotion of this invention include urea itself as well as the mono-, di-, tri-, and tetra-lower alkyl-substituted derivatives. The phrase "lower alkyl" is used herein to include alkyl groups that contain 1-4 carbon atoms. Thus, substituted ureas such as N-ethylurea, N,N'-dimethylurea, N-butyl-N'-propylurea, N,N,N'-trimethylurea, N,N,N',N'-tetraethylurea, and the like are contemplated, as are mixtures of the substituted ureas with themselves and with urea itself. Urea, itself, is most preferred herein and will be used illustratively hereinafter.

Urea is preferably used in the activator of this invention at a concentration of about 0.5 to about 12 molar, or at about 3 to about 65 weight percent. In more preferred practice, the urea is

-7-

present at about 6 to about 55 weight percent or about 1 to about 11 molar, and most preferably at about 40 to about 55 weight percent or about 8 to about 11 molar.

The activator also includes an ammonium salt of a strong acid; i.e., an acid having a $pK_a$ value of about 1 or less. Exemplary strong acids include hydrochloric, sulfuric, benzenesulfonic and pyrophosphoric acids. Particularly preferred ammonium salts of strong acids are ammonium chloride, ammonium sulfate, and mixtures thereof.

The amount of the ammonium salt of a strong acid (hereinafter "ammonium salt") useful to provide the unexpected stability to the aqueous urea activator varies with the amount of urea in the activator. One convenient way to measure the amount of ammonium salt needed is by the pH value of the resulting solution after the addition of the ammonium salt. Thus, it has been found that when an ammonium salt has been added to the aqueous urea solution in an amount sufficient to adjust the pH value of that solution to about 6 to about 8, the urea in the resulting solution is stabilized to the production of titratable alkali. More preferably, an amount of ammonium salt of a strong acid is utilized that is sufficient to adjust the urea solution of the activator to a pH value of about 6.5 to about 7.5.

Quantitatively, the amount of ammonium salt useful for adjusting the pH value of an activator of this invention is about 0.02 to about 1 molar ($\underline{M}$). More preferably, the ammonium salt is present at about 0.05 to about 0.75 $\underline{M}$ in the activator.

As can be seen from the graph of the Figure, there is not a straight line relationship between the molar concentration of ammonium ion and the change in

-8-

pH value for the amount of urea shown. Nevertheless, it can be said that when more urea is present in the activator solution, more ammonium salt is required to adjust the pH value to the desired range to obtain stabilization. The presence of less urea requires a smaller amount of ammonium salt to provide pH value adjustment and stability. The use of less urea in an activator with a constant amount of an ammonium salt generally provides a lower pH value. This can be seen for the points of the graph that correspond to a 20 weight percent urea solution.

The mechanism by which the aqueous urea is stabilized by the ammonium salt is not understood. The improved stability is not believed to be due simply to the near neutral pH value of the urea-ammonium salt activator, because as is illustrated in Tables 1 and 2 of Example 1, solutions having the same amount of urea and that are within the pH value range of the activator herein are not stable.

It is believed that increased ionic strength or molality caused by the addition of the ammonium salt does not cause the observed stability of urea since a similar amount of sodium chloride does not lead to a relative decrease in the production of titratable alkali. This is shown in Table 2 of Example 1.

A particular utility for the activator of this invention is as a component of a hair waving system that is utilized to prepare a lotion for reductive hair curling (waving) and straightening. Since the chemistry involved in hair curling and straightening is substantially similar, both processes will be referred to hereinafter as "waving".

The word "reductive" is meant herein to differentiate the present method of waving from those hair treating processes that are carried out at high pH values, e.g. at about pH 11 to about pH 14. The high pH treatments occur primarily by the transformation of disulfide bonds in hair keratin cystine groups into lanthionine groups, while the reductive treatments are believed to occur by breaking and then reforming the hair keratin disulfide bonds.

The activator of this invention comprises a separately packaged component of a hair waving system that is admixed with at least a second, separately packaged component of the hair waving system to form the waving lotion. The second separately packaged component of the hair waving system is termed a base lotion, and includes a water-soluble reducing agent, such as a thiol or bisulfite ion, as a reductive hair disulfide bond-breaking agent.

Exemplary water-soluble thiol-containing reducing agents for use in a second, separately packaged component of the hair waving system include mercaptocarboxylic acids, their alkali metal and ammonium salts, their esters and particularly glyceryl esters, and mixtures thereof. Examples of these reducing agents include thioglycolic acid, thiolactic acid, 3-mercaptopropionic acid and the like. Suitable alkali metal salts of these acids include sodium or potassium salts. Ammonium salts can also be used, and include salts prepared from these acids and ammonia, mono- di- or triethanolamine and mono-, di- or tri-iso-propanolamine. Particularly preferred mercaptocarboxylic acid esters include glyceryl thioglycolate.

-10-

Ammonium thioglycolate is a particularly preferred thioglycolate salt although alkali metal thioglycolates such as sodium thioglycolate and hydroxy lower alkyl substituted-ammonium salts such as 2-hydroxyethylammonium thioglycolate are also useful. Upon admixture prior to use, the base lotion supplies the water-soluble salt of thioglycolic acid to the waving lotion so formed. The admixed waving lotion preferably contains about 4 to about 20 weight percent water-soluble salt of thioglycolic acid, and more preferably at about 8 to about 17 weight percent, calculated as thioglycolic acid.

Exemplary, water-soluble bisulfite ion-containing reducing agents include alkali metal and ammonium bisulfite. The cleavage of hair disulfide bonds using the bisulfite ion in waving processes is sometimes referred to as "sulfitolysis." Since bisulfite ion is a well known reducing agent, and since its action upon the hair includes production of hair keratin thiol groups which remain in the hair during waving, bisulfite ion is included herein as a reducing agent.

The hair disulfide bond-breaking agent is present in the base lotion in an amount such that, after admixture with the activator, an amount sufficient to wave hair is present in the waving lotion so produced.

Thus, when the particularly preferred thiol-containing acids or acid salts are utilized, they are preferably present at an amount to provide about 1 to about 20 weight percent of the waving lotion, measured as the free acid. Thiol-containing esters, such as glyceryl thioglycolate, are particularly preferred to be utilized at about 3 to about 30 weight percent of the waving lotion. Alkali

-11-

metal or ammonium bisulfites are preferably present in the waving lotion at from about 5 to about 15 weight percent, and more preferably from about 10 to about 13 weight percent.

The pH value of the base lotion is sufficient to provide a pH value of about 6.5 to about 9.5 to the waving lotion after admixture with the activator. That is, the base lotion has a pH value and contains a sufficient amount of acidic or basic pH value-adjusting agent to adjust the pH value of the admixed waving lotion to be about 6.5 to about 9.5. More preferably, the base lotion pH value is sufficient to provide a pH value of about 6.5 to about 8 to the waving lotion, and most preferably, for a thioglycolate-containing waving lotion, the base lotion provides a waving lotion pH value of about 6.5 to about 7.5.

In a usual waving process the hair is typically first shampooed and towel blotted. The waving lotion is applied and maintained in contact with the hair for a period of time sufficient to form partially reduced hair; i.e., to break enough of the hair keratin disulfide bonds to produce a change in configuration if that hair is wound on rollers and then neutralized. The amount of time required to produce a permanent wave using the above described compositions and agents is well known in the art.

After the partially reduced hair is formed, the waving lotion is substantially rinsed from the partially reduced hair. In usual practice, about three minutes of rinsing in a sink using running warm tap water is sufficient to rinse the hair substantially free of the first, reducing agent-containing, composition. As used herein, the term "rinse" in any of its grammatical forms includes treatment with a shampoo.

-12-

The hair so treated is usually strained by wrapping it upon rollers of a diameter chosen to impart an increased or decreased amount of curl. Wrapping of the hair on the rollers can be accomplished at any time prior to the neutralization step in which the disulfide bonds are reformed. The treated hair can also be waved without the use of rollers so that the weight of the wet hair provides straightening strain in what is called "straight waving." Wrapping on rollers or straight waving changes the configuration of the partially reduced hair.

The final step required in waving is neutralization of the partially reduced hair by the application of a neutralizing composition to contact the hair and rebuild the broken hair keratin disulfide bonds to form the waved hair. Several agents for rebuilding hair keratin disulfide bonds which have been cleaved by a thioglycolate salt are known in the art. These agents are typically oxidizing agents such as hydrogen peroxide, perborate or bromate salts, such as sodium perborate and sodium bromate.

When bisulfite ion is utilized as a reductive hair disulfide bond-breaking agent, it is well known that the hair can be neutralized and the hair keratin disulfide bonds remade by treating the partially reduced hair with an aqueous composition having a pH value of about 8.5 to about 10.5. Several agents for rebuilding hair keratin disulfide bonds which have been cleaved by a bisulfite salt are known in the art. Typically, these agents are water-soluble alkalizing agents such as sodium carbonate and sodium bicarbonate. Other agents such as water-soluble disulfide salts like diammonium

-13-

dithiodiglycolate can also be used to reform the hair disulfide bonds from partially reduced hair prepared using bisulfite ion, as can oxidizing agents, such as hydrogen peroxide.

The concentration of agents within the neutralizing composition and the pH value of that composition are well known to those skilled in the hair waving arts and need not be discussed fully herein. However, when a waving lotion is utilized containing a thioglycolate salt as the hair disulfide bond-breaking agent, an aqueous hydrogen peroxide solution is preferred for rebuilding the hair disulfide bonds. The hydrogen peroxide is preferably used at a concentration of about 1 to about 3 weight percent and at a pH value from about 3 to about 6.

A hair waving product that includes the waving lotion system and at least a separately packaged neutralizer to reform the broken disulfide bonds as described above is also contemplated in this invention.

The present invention also contemplates a particular waving lotion, regardless of its method of formation. The waving lotion of this invention utilizes an ammonium, hydroxy lower alkyl substituted-ammonium or alkali metal salt of thioglycolic acid (thioglycolate salt), urea and an ammonium salt of a strong acid. This waving lotion has a pH value of about 6.5 to about 8, and provides an improved waving result compared to that of a similar composition at the same pH value that contains no ammonium salt of a strong acid.

This waving lotion contains (a) the thioglycolate salt at a concentration of about 4 to about 20 weight percent, calculated as thioglycolic acid, and more preferably at about 8 to about 17

-14-

weight percent, (b) urea at about 4 to about 25 weight percent, and more preferably at about 8 to about 17 weight percent, and (c) an ammonium salt of a strong acid present at about 0.1 to about 2 weight percent (about 0.02 to about 0.4 molar) and more preferably at about 0.1 to about 1 weight percent. The preferred pH value range for this waving lotion is about 6.5 to about 8, more preferably about 6.5 to about 7.5.

The waving lotion of this invention can also include about zero to about 10 weight percent ethanol to assist in penetration of the hair fiber, zero to about 1 weight percent ammonium bicarbonate to adjust the pH value, perfume, colorant, conditioners and the like as are usually found in waving lotions.

The pH value of a waving lotion of this invention is unusually low for waving with a thioglycolate salt as compared to waving lotions known in the art for usual use with thioglycolate salts. As noted, a thioglycolate salt-containing waving lotion of this invention has a pH value of about 6.5 to about 8. That range of pH value is to be compared to the usual ammonium thioglycolate waving pH value of about 8.5 to about 9.5.

It was quite unexpected that the presence of the relatively small amount of ammonium salt used in the waving lotion of this invention would provide the substantial improvements in curl level and curl spring that have been observed. An illustration of these improvements is shown in Example 2.

The improvement in curl level was additionally unexpected in view of the teachings of U.S. Patent No. 2,717,228. That patent teaches the use of similar amounts of urea and thioglycolate salt

-15-

to those utilized herein in waving lotions having pH values in the higher range of 8.6 to 9.5. That patent also teaches that ammonium salts of strong acids such as those used herein deswell the hair and thus help to retard over processing, which is not a significant problem at the pH values of the present waving lotion. The amount of ammonium ion taught as useful in that patent is generally greater than the amount used herein, but nevertheless overlaps with the concentrations found to be useful in this invention.

Lotions No. 2, 4 and 5 of Table III of U.S. Patent No. 2,717,228 illustrate the wave rating (level) of three compositions, each of which contained 1.5 molar urea (9 weight percent). Examination of the data for Lotions No. 2 and No. 4 shows that the wave rating was the same when waving was carried out with a waving lotion having a pH value of 9.3 and either no ammonium salt was present (Lotion No. 2) or 1.0 molar ammonium ion was present (Lotion No. 4). Examination of data for Lotion No. 5 shows that a lower wave rating was obtained when 0.6 molar ammonium ion was used at a waving lotion pH value of 9.1. The data for waving lotion No. 6 in Table III show that the same, low wave level was maintained at a lotion pH value of 9.0 with 0.6 molar ammonium ion even when the urea concentration was raised to 2 molar (12 weight percent).

Thus, the data in Table III of U.S. Patent No. 2,717,228 indicate that for high pH (above about pH 9) waving lotions that contain urea, wave rating or curl level decreases with decreasing pH value regardless of the presence of a relatively high, but decreasing amount of ammonium salt of a strong acid. Those results also show a constant wave rating at a

-16-

given, elevated pH value whether the ammonium salt is present or not. On the other hand, the results illustrated in Example 2 for the urea-containing, lower pH (below pH 8) waving lotion of this invention show that the presence of a relatively small amount of ammonium salt of a strong acid is quite beneficial to improving the curl level and curl spring.

As noted hereinbefore, the present invention contemplates related developments. Included among those developments are (1) a waving lotion, and (2) a two-package hair waving system that can form the waving lotion (1) when the contents of the two packages are combined.

The waving lotion (1) need not, however, be made from the two-package hair waving system (2). Rather, the ammonium salt of a strong acid and the ammonium thioglycolate of the waving lotion of this invention can be packaged together and the urea packaged in dry form if it is so desired. Any other convenient manner of packaging can also be used. It is, however, preferred that the waving lotion of this invention be prepared using the hair waving system of this invention.

When utilized as part of a hair waving system of this invention the activator is included in one of at least two separately packaged components. A second of the separately packaged components includes an aqueous solution of a reductive, hair disulfide bond-breaking agent. The separately packaged components are admixed to form the waving lotion which is then applied to the hair. An exemplary waving lotion so produced contains urea, an ammonium salt of a strong acid and the thioglycolate in the amounts and at the pH values described hereinbefore.

-17-

After admixture of the separately packaged components of the hair waving system to form the waving lotion, that lotion is applied to the hair to contact the hair fibers, and maintained in contact with the hair fibers to form partially reduced hair that contains broken disulfide bonds in the manner known in the art. Upon formation of the partially reduced hair, hair is strained and then rinsed to wash the waving lotion from the hair. The hair is then neutralized by a method also well known in the art to reform the disulfide bonds within the hair.

Additional, separately packaged components may also be included with the hair waving system and/or the hair waving product of this invention. The additional component may also be included within either package of the hair waving system.

An example of such an additional component is a polymeric conditioner such as the material formed by the reaction of dimethyl sulfate and a copolymer of vinylpyrrolidone and dimethylaminoethyl methacrylate sold under the trademarks GAFQUAT 734 and GAFQUAT 755 by GAF Corporation. A more preferred polymeric conditioner is the polymer of hydroxyethyl cellulose reacted with epichlorohydrin and then quaternarized with trimethylamine that is sold under the trademarks JR-30M and JR-125 by Union Carbide Corporation.

Weight percentages of the various ingredients discussed and claimed herein are given based upon the whole weight of a composition.

This invention is further illustrated by the Examples that follow.

-18-

## Best Mode For Carrying Out the Invention

Example 1.    Retardation of Production of Titratable Alkali in Solutions That Contain Urea

The retardation of the production of titratable alkali, and presumed urea hydrolysis, was determined for aqueous solutions that contained 50 weight percent urea, alone or with additives. The number of milliequivalents (meq.) of free alkali per milliliter (ml) of solution was determined after storage by titration of a 10 ml urea solution with 1 normal (N) sulfuric acid to the methyl red endpoint (pH 5.8-6.0) using the following equation:

Meq. of free alkali/ml of urea solution =

$$\frac{\text{ml of } 1N \ H_2SO_4}{10 \ ml}$$

The urea solutions, without additives, had pH values ranging from 8.8 to 9.8 when prepared in deionized water. Addition of the additives adjusted the pH values of those urea solutions to values of 6.5 to 7.9. The solutions for which the free alkali determinations were made were aged at a temperature of 43°C. (110°F.) for up to 8.5 months or at a temperature of 60°C. (140°F.) for up to 9 weeks. The results of the determinations are given in Tables 1 and 2, before, for aging at 43°C. and 60°C, respectively.

-19-

Table 1

Production of Alkali From Urea

Solutions Aged at 43°C.

| Additive In Urea Solution[1] | Initial pH Value | Aging Time (Months) | Meq.Alkali/ml Urea Solution | |
|---|---|---|---|---|
| | | | Initial | After Aging |
| None | 9.8 | 1.5 | 0.005 | 0.07 |
| None | 9.3 | 3.5 | 0.005 | 0.28 |
| None | 9.3 | 7 | 0.005 | 0.50 |
| None | 8.8 | 8.5 | 0.003 | 0.62 |
| Ammonium chloride (0.5M) | 7 | 5 | 0.005 | 0.02 |
| HEEDTA[2] (0.1 wt.%) | 7.2 | 4.5 | 0.005 | 0.24 |
| HEEDTA[2] + Ammonium chloride (0.1 wt.% + 0.19M, respectively) | 7.2 | 4.5 | 0.005 | 0.04 |
| Citric acid (0.01 wt.%) | 6.5 | 8.5 | 0.000 | 0.60 |
| Citric acid (0.005 wt.%) | 7.5 | 8.5 | 0.000 | 0.61 |

Note 1: All solutions contained 50 weight percent urea in deionized water.

Note 2: HEEDTA is N-hydroxyethylethlene-diaminetriacetic acid sold under the trademark HAMP-OL ACID by W.R. Grace. Addition of HEEDTA in the amounts shown brought the pH value of both solutions in which it was used to about 5.6. The pH value of the solutions was adjusted to 7.2 by the addition of ammonium hydroxide.

-20-
## Table 2
### Production of Alkali From Urea Solutions Aged at 60°C.

| Additive In Urea Solution[1] | Initial pH Value | Meq. Alkali/ml Urea Solution at Various Times | | |
|---|---|---|---|---|
| | | Initial | 3 Weeks | 9 Weeks |
| None | 9.5 | 0.005 | 0.40 | 1.13 |
| Ammonium Chloride (0.14M) | 7.9 | 0.005 | 0.17 | 0.66 |
| Ammonium Chloride (0.22M) | 7.1 | 0.005 | 0.10 | 0.38 |
| Ammonium Sulfate (0.23M ammonium ion) | 7.3 | 0.005 | 0.06 | 0.15 |
| Sodium chloride (0.26M) | 9.0 | 0.005 | 0.46 | 1.17 |
| Citric acid (0.005 wt.%) | 7.5 | 0.005 | 0.47 | 1.23 |
| Citric acid (0.01 wt.%) | 6.5 | 0.000 | 0.43 | 1.23 |

Note 1: All solutions contained 50 weight percent urea in deionized water.

The data in the above Tables illustrate that the ammonium chloride and ammonium sulfate retard the production of titratable alkali from the urea solutions. The data also show that the reduction of titratable alkali is not merely a result of the lowered pH value caused by the ammonium salt in that addition of citric acid to adjust the solution pH value to the range obtained by addition of ammonium salt did not retard production of alkali, but actually increased that production.

-21-

HEEDTA, a sequestering agent, was added in small amount, 0.1 weight percent, to a urea solution to retard urea decomposition that might be caused by metal ion catalysis. The results using HEEDTA alone were equivocal as to protection from metal ion catalysis.

The production of titratable alkali was substantially reduced when the same amount of HEEDTA and 0.19 molar ammonium chloride, a particularly preferred ammonium salt, were admixed together with the same amount of urea and at the same pH value as had been used in the determination utilizing HEEDTA alone.

The solutions containing sodium chloride produced slightly more titratable alkali at a slightly lower pH value than was produced in the solution containing urea alone. Yet, the concentration of sodium chloride was about the same as the concentrations of ammonium ion. These facts are believed to indicate that the ammonium salts useful herein do not reduce the production of titratable alkali by increasing the ionic strength of the urea solutions.

Improvement Of Low pH Waving
With Waving Lotions Containing
Thioglycolate And Urea By
Including An Ammonium Salt Of
Example 2:  A Strong Acid

Wave levels were determined and compared on models having normal hair, i.e. hair having no history of bleaching, waving or tinting, as well as on models having tinted hair. Four models having each type of hair were used.

One-half of each model's hair was waved with a waving lotion of this invention (Composition A) that was similar to the waving lotion prepared in Example 3, and was prepared from a waving lotion

-22-

system similar to that of Example 3. The other half of the hair was waved with a waving lotion not of this invention that contained the same amounts of ammonium thioglycolate and urea, but no ammonium salt of a strong acid (Composition B).

Composition A of this invention contained ammonium thioglycolate in an amount of 12 weight percent calculated as thioglycolic acid, 12.5 weight percent urea and 0.06 molar ammonium chloride and had a pH value of 7.1. Composition B was identical to Composition A except that water replaced ammonium chloride.

Except for the compositions used, the treatments for both sides of each model's hair was identical. Thus, each model's hair was shampooed, towel blotted and wound on curlers. The wound hair on each model was saturated with either Composition A or Composition B. The hair was thereafter covered with a plastic cap and processed under a pre-heated, salon-type hair dryer for 20 minutes for normal hair or 10 minutes for tinted hair. The hair was rinsed, blotted and neutralized with a solution of hydrogen peroxide (2.2 weight percent at pH 3.5) for a time period of 5 minutes. The curls were taken down, and rinsed.

Curl level and curl spring of the wet hair was rated by at least two experienced evaluators who did not know which sides of each head had been treated with the waving lotion of this invention. The evaluations were conducted on both the day of waving (DW), and at a one week (1W) return subsequent to a shampoo.

The evaluators noted for every model that the side of the head treated with the composition of this invention had a noticably higher curl level and

-23-

curl spring than did the hair on the other side. Average ratings from these evaluations are listed in Table 3.

Table 3

Curl Level and Spring Ratings

| Hair Type | Composition A | | Composition B | |
|-----------|---------------|-----|---------------|-----|
|  | DW[1] | 1W[2] | DW[1] | 1W[2] |
| Curl Level[3] | | | | |
| Normal | 8.5 | 8.3 | 7.6 | 7.0 |
| Tinted | 10 | 8.8 | 9.3 | 8.0 |
| Curl Spring[4] | | | | |
| Normal | 3.8 | 3.0 | 3.3 | 2.8 |
| Tinted | 4.8 | 3.8 | 4.0 | 3.1 |

Note 1: DW - Day of wave

Note 2: 1W - One week return.

Note 3: Curl level is rated on a scale of 1 to 10; 10 being best.

Note 4: Curl spring is rated on a scale of 1 to 5; 5 being best.

The unexpectedly improved amounts of curl level and curl spring that are effected by using of a waving lotion of this invention are amply demonstrated by the data of Table 3. It is noted that the data show no reversals where the waving lotion of this invention did not show an improvement over the lotion with which it was compared.

Example 3: Waving Lotion System

A waving lotion system of this invention was prepared containing a separately packaged activator and a separately packaged base lotion including a salt of thioglycolic acid for reductively breaking disulfide bonds in hair.

-24-

The activator was prepared as follows:

| Ingredient | Weight Percent |
|---|---|
| 1. Deionized water | 48.8 |
| 2. Urea (prilled) | 50.0 |
| 3. Ammonium chloride | 1.2 |
| | 100.0 |

The water was heated to a temperature of 60° C. (about 140° F.) and the urea was added with agitation to provide a solution. The resulting solution was reheated to a temperature of 40° C. (about 105° F.) and the ammonium chloride was admixed. The resulting admixture was agitated until a substantially homogeneous, clear, colorless activator solution resulted which had a pH value of about 6.9-7.3 over several preparations.

The aqeuous base lotion containing the thioglycolate salt was prepared as follows:

| Ingredient | Weight Percent |
|---|---|
| 1. Deionized water | 71.53 |
| 2. Ammonium thiglycolate (60 perent active as thioglycolic acid) | 26.67 |
| 3. Polyoxyethylene (15) nonyl phenyl ether | 0.88 |
| 4. Perfume | 0.22 |
| 5. Ammonium hydroxide (28%) to about pH 7.1 | 0.70 |
| | 100.00 |

Ingredients 1 and 2 were admixed until substantially homogeneous. A premixture of ingredients 3 and 4 was prepared and then added to the admixture formed from ingredients 1 and 2 with stirring to form a substantially homogeneous resulting admixture. Ingredient 5 was thereafter added slowly with agitation and the pH value of the composition was adjusted to 6.9-7.3 to provide a clear, colorless solution of disulfide bond-breaking agent.

Each of the compositions so prepared was packaged separately in plastic containers to provide a waving lotion system on admixing. The package of the activator contained 27.5 grams of that composition. The second bottle which contained the base lotion, including ammonium thioglycolate, contained 82.5 grams of its composition.

Admixture of the contents of both of the above bottles provided a clear, colorless waving lotion system of this invention that contained about 11.8 to about 12.2 weight percent ammonium thioglycolate, calculated as thioglycolic acid, about 12 to about 12.5 weight percent urea and about 0.29 to about 0.3 weight percent ammonium chloride. The pH value of the waving lotion averaged about 6.9 to about 7.3 over several preparations.

Storage of another package of the same activator at 110°F. (about 43°C.) for 4.5 months showed a change in titratable alkali from an initial value of about 0.005 milliequivalents per milliliter to about 0.02 milliequivalents per milliliter. Thus, the stability imparted by the ammonium chloride was again demonstrated.

Use of the waving system and waving lotion prepared in this Example on dozens of models' hair provided excellent waving results on both tinted and normal hair.

Hair Property Study After Waving Example 4:    Tresses With Ammonium Thioglycolate

The condition of hair treated with an prewrap composition that contained ammonium bisulfite as the prewrap agent pursuant to U.S. Patent No. 4,214,596 to Kaplan et al. and then waved with the waving lotion of Example 3 (Treatment A) was compared to that of hair waved with a commercially available, alkaline thioglycolate waving lotion (Treatment B). Untreated hair was used as the control (Treatment C). The commercially available alkaline waving lotion contained no ammonium salt of a strong acid, nor urea. It is noted that the prewrap treatment breaks no more than about 5% of the hair disulfide. bonds and therefore does not form partially reduced hair.

The "liquid retention" method discussed by Wolfram and Underwood in Textile Research Journal, 36(11), pp. 947-953 (1966) was utilized for the comparison, with distilled water replacing the buffer used in the above article, a 15-minute soaking time and centrifugation for 30 minutes. Liquid retention determinations reflect the condition of the hair measured by the porosity of the fibers.

Hair tresses of normal brown hair (DeMeo Brothers, New York) were shampooed, water rinsed, and then blotted with a towel. A tress was treated with the prewrap lotion by applying the lotion onto the hair, followed by combing and rolling the tress onto a conventional curler. The waving lotion prepared from a hair waving system similar to that of Example 3 was then applied to the prewrap-treated hair tress. This waving lotion contained 10 percent urea and 0.1 percent ammonium chloride. The treated tress was covered with a plastic cap and processed under a pre-heated salon-type hair dryer for 20 minutes. The prewrap lotion and waving lotion were then rinsed from the hair and the rolled hair was neutralized in the usual manner using a commercial neutralizer solution containing hydrogen peroxide. The remaining tress was waved with a commercialy available alkaline thioglycolate lotion using a conventional cold-waving process; i.e., no prewrap lotion and no heat were used in the procedure.

The liquid retention results are shown in Table 4.

Table 4

Liquid Retention of Waved Tresses

| Treatment | Percent Liquid Retention |
|---|---|
| A (pH 6.9-7.2) | 42.6 |
| B (pH 9.3-9.5) | 51.8 |
| C (Control) | 36.0 |

The data in the above Table illustrate that a thioglycolate-containing waving lotion of this invention (Treatment A) prepared from a waving lotion system of this invention left the hair in better condition than did commercially available alkaline waving lotion. That improved hair condition was noted even though a prewrap and elevated temperature were used, either condition alone being capable of increasing the liquid retention value with an alkaline thioglycolate waving lotion.

The present invention has been described with respect to preferred embodiments. It will be clear to those skilled in the art that modifications

-28-

and/or variations of the disclosed compositions and method can be made without departing from the scope of the invention set forth herein. The invention is defined by the claims that follow.

# C L A I M S

1. A hair waving system comprising an activator and a base lotion as separately packaged components that are admixed prior to use to form a waving lotion,

said activator including water, a urea compound selected from the group consisting of urea, lower alkyl substituted urea and mixtures thereof present at about 0.5 to about 12 molar, and a sufficient amount of an ammonium salt of a strong acid to adjust the pH value of said activator to about 6 to about 8; and

said base lotion including water, and a water-soluble reductive hair disulfide bond-breaking agent, said bond-breaking agent present in sufficient amount to wave hair after admixture with said activator, said base lotion having a pH value sufficient to provide a pH value of about 6.5 to about 9.5 to the waving lotion after admixture with said activator.

2. A hair waving system comprising an activator and a base lotion as separately packaged components that are admixed prior to use to form a waving lotion,

said activator including water, a urea compound selected from the group consisting of urea, lower alkyl substituted urea and mixtures thereof present at about 0.5 to about 12 molar, and a sufficient amount of an ammonium salt of a strong acid to adjust the pH value of said activator to about 6 to about 8; and

said base lotion including water, and a water-soluble salt of thioglycolic acid present in an amount sufficient to supply about 4 to about 20 weight percent thioglycolic acid to said waving lotion after admixture with said activator, said base lotion having a pH value sufficient to provide a pH value of about 6.5 to about 8 to the waving lotion after admixture with said activator.

3. The hair waving system according to claim 2 additionally including a separately packaged neutralizer to reform broken disulfide bonds in hair.

4. A hair waving system comprising an activator and a base lotion as separately packaged components that are admixed prior to use to form a waving lotion;

said activator including water, urea present at about 1 to about 11 molar, and an ammonium salt of a strong acid present at about 0.02 to about 1 molar, said activator having a pH value of about 6 to about 8; and

said base lotion including water, ammonium thioglycolate present in an amount sufficient to supply about 4 to about 20 weight percent thioglycolic acid to said waving lotion after admixture with said activator,

said base lotion having a pH value sufficient to provide a pH value of about 6.5 to about 8.0 to said waving lotion after admixture with said activator.

5. A hair waving lotion comprising water, a water-soluble salt of thioglycolic acid present at about 4 to about 20 weight percent calculated as thioglycolic acid, a urea compound selected from the group consisting of urea, lower alkyl substituted urea and mixtures thereof present at about 4 to 25 weight percent, and an ammonium salt of a strong acid present at about 0.1 to about 2 weight percent, said waving lotion having a pH value of about 6.5 to about 8.

6. A hair waving lotion comprising water, a water-soluble salt of thioglycolic acid present at about 8 to about 17 weight percent calculated as thioglycolic acid, about 4 to about 25 weight percent urea, and about 0.1 to about 2 weight percent of an ammonium salt of a strong acid, the pH value of said waving lotion being about 6.5 to about 7.5.

7. A method of waving hair comprising the steps of:

   providing the hair waving system of claim 1;
   admixing said activator and said base lotion to form a waving lotion;
   contacting said waving lotion to hair;
   maintaining said contact for a time sufficient to form partially reduced hair containing broken disulfide bonds;
   straining said partially reduced hair; and
   applying a neutralizer to the strained, partially reduced hair to reform the disulfide bonds.

8. A method of waving hair comprising the steps of:

   providing the hair waving system of claim 2;
   admixing said activator and said base lotion to form a waving lotion;
   contacting said waving lotion to hair;
   maintaining said contact for a time sufficient to form partially reduced hair containing broken disulfide bonds;
   straining said partially reduced hair; and
   applying a neutralizer to the strained partially reduced hair to reform the disulfide bonds.

9. A method of waving hair comprising the steps of:

   providing the waving lotion of claim 23;
   contacting said waving lotion to hair;

maintaining said contact for a time sufficient to form partially reduced hair containing broken disulfide bonds;

straining said partially reduced hair; and applying a neutralizer to the strained, partially reduced hair to reform the disulfide bonds.

10. A method of waving hair comprising the steps of:

contacting the hair with an aqueous prewrap agent-containing solution and maintaining said contact for a period of time sufficient to break less than about 5 percent of the hair disulfide bonds;

providing the waving lotion of claim 5; contacting said waving lotion with said prewrap-contacted hair;

maintaining said waving lotion contact for a time sufficient to form partially reduced hair containing broken disulfide bonds;

straining said partially reduced hair; and applying a neutralizer to the strained, partially reduced hair to reform the disulfide bonds.

PH VALUES OF AQUEOUS UREA SOLUTIONS AS A FUNCTION OF REAGENT CONCENTRATION

△ = AMMONIUM CHLORIDE (50% UREA)

⬡ = AMMONIUM CHLORIDE (20% UREA)

● = AMMONIUM SULFATE (50% UREA)

▼ = SODIUM CHLORIDE (50% UREA)

PH OF UREA SOLUTION

MOLARITY OF REAGENT CATION

1/1